(19) Europäisches Patentamt
European Patent Office
Office européen des brevets

(11) Publication number: 0 018 803
B1

(12) EUROPEAN PATENT SPECIFICATION

(45) Date of publication of patent specification: 12.05.82

(21) Application number: 80301373.9

(22) Date of filing: 25.04.80

(51) Int. Cl.³: C 07 C 178/00, C 07 C 179/035, C 07 C 37/08

# (54) Process for preparation of aromatic tertiary hydroperoxides and phenols prepared therefrom.

(30) Priority: 27.04.79 JP 51537/79

(43) Date of publication of application: 12.11.80 Bulletin 80/23

(45) Publication of the grant of the patent: 12.05.82 Bulletin 82/19

(84) Designated Contracting States: DE FR GB IT NL

(56) References cited:
GB - A - 745 128
US - A - 4 013 725

HOUBEN-WEYL "Methoden der organischen Chemie" 4th edition, vol. VI/1c, part 1, 1976, GEORG THIEME VERLAG, Stuttgart.

(73) Proprietor: MITSUI PETROCHEMICAL INDUSTRIES, LTD.
2-5, Kasumigaseki 3-chome Chiyoda-ku
Tokyo 100 (JP)

(72) Inventor: Matsunaga, Fujihisa
2-5, 1-chome
Muronoki-cho, Iwakuni-shi, Yamaguchi-ken (JP)
Inventor: Shinohara, Yoshiyuki
18-5, 2-chome
Fuseshin-machi, Kashiwashi, Chiba-ken (JP)
Inventor: Okubo, Isamu
12-8, 2-chome
Shi-machi, Ohtake-shi, Hiroshima-ken (JP)
Inventor: Nakamura, Takayuki
2-9, 1-chome, Muronoki-cho
Iwakuni-shi, Yamaguchi-ken (JP)
Inventor: Tanaka, Seiichi
12-6, 2-chome
Shin-machi, Ohtake-shi, Hiroshima-ken (JP)

(74) Representative: Myerscough, Philip Boyd et al,
J.A.Kemp & Co. 14, South Square Gray's Inn
London, WC1R 5EU (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 018 803 B1

# Process for preparation of aromatic tertiary hydroperoxides and phenols prepared therefrom

The present invention relates to a process for the preparation of aromatic tertiary hydroperoxides and the phenols prepared therefrom.

The preparation of aromatic hydroperoxides by subjecting an aromatic compound having a secondary alkyl group, such as cumene, cymene, dimethylcumene, diisopropylbenzene or sec-butylbenzene, to liquid phase oxidation with a molecular oxygen-containing gas in the presence of a basic aqueous solution is well known. This process is used industrially for the preparation of precursors of phenols. Also the preparation of aromatic hydroperoxides by subjecting an aromatic compound having a secondary alkyl group, such as mentioned above, to liquid phase oxidation with a molecular oxygen-containing gas in the absence of a basic aqueous solution is known.

Since the rate of liquid phase oxidation is low in these known processes, it has been proposed to increase the oxidation rate by employing as a catalyst a compound of a heavy metal such as copper, manganese, cobalt, nickel or iron (see British Patent No. 665,897, British Patent No. 665,898, British Patent No. 745,128, British Patent No. 760,367, British Patent No. 801,387, U.S. Patent No. 2,954,405, Japanese Patent Publications Nos. 36899/70, 28604/70, 6568/71 and 7148/74, and Japanese Patent Application Laid-Open Specifications Nos. 507/73 and 72225/74). However, in each of these processes, the rate of the liquid phase catalytic oxidation is not sufficiently high. When the aromatic compound to be oxidized is an aromatic compound having a primary alkyl group as well as a secondary alkyl group, such as cymene, dimethylcumene or diisopropoyltoluene, the oxidation rate is much lower than in case of an aromatic compound having a secondary alkyl group alone, and furthermore, a primary aromatic hydroperoxide or secondary aromatic hydroperoxide is competitively formed as the oxidation product in addition to an intended tertiary aromatic hydroperoxide, resulting in reduction of the selectivity of the oxidation reaction.

As a means for improving selectivity, in the liquid phase catalytic oxidation of an aromatic compound having a secondary alkyl group and a primary alkyl group, it has been proposed to effect the liquid phase catalytic oxidation in the presence of a heavy metal compound catalyst and an aqueous phase by using a molecular oxygen-containing gas (see Japanese Patent Application Laid-Open Specifications Nos. 142526/75 and No. 142527/75). According to these proposals, especially the latter Laid-Open Specification, a chelate compound of a heavy metal is used as the catalyst and the liquid phase catalytic reaction is carried out while adjusting the pH value of the aqueous phase to a value in the weakly acidic or neutral region, that is to a pH of 5 to 7.5. The selectivity is improved to some extent by this technique. However decomposition of the aromatic hydroperoxide occurs to a noticeable degree in the catalytic oxidation system and phenol compounds are formed as by-products. Since these phenol compounds rather inhibit the oxidation reaction, there is a reduction in the oxidation rate.

It has now been found that the problems of the oxidation rate and selectivity may be overcome by using an inorganic salt or organic acid salt of copper as a catalyst and by maintaining the catalyst concentration in the liquid phases and the pH value of the basic aqueous phase at specific levels.

Accordingly, the present invention provides a process for the preparation of an aromatic tertiary hydroperoxide by contacting an oily liquid phase comprising an aromatic compound having a secondary alkyl group, which phase is being agitated with a basic aqueous liquid phase, with a molecular oxygen-containing gas in the presence of a metal compound catalyst, thereby oxidizing the aromatic compound to a corresponding aromatic tertiary, hydroperoxide, wherein the metal compound catalyst is a copper compound catalyst composed of an inorganic salt or organic acid salt of copper, and during oxidation the total concentration of the copper compound catalyst in the two phases is maintained at from 0.005 to 10 ppm, calculated on the basis of copper in the catalyst and the pH value of the basic aqueous phase is maintained at from 7.6 to 10.

By adopting such a process not only can the oxidation rate be improved over rates attainable by the conventional process using an oxidation catalyst, but also the tertiary aromatic hydroperoxide may be prepared selectively in a high yield even if an aromatic compound having a primary alkyl group as well as a secondary alkyl group is used as a starting compound.

In the present invention, an aromatic compound having a secondary alkyl group is used as the compound to be oxidized. This aromatic compound may have a substituent which does not inhibit the liquid phase catalytic oxidation reaction in addition to the secondary alkyl group. The aromatic compound may be, for example, cumene, cymenes such as o-cymene, m-cymene, p-cymene and a mixture of cymene isomers, dimethylcumenes such as 3,5-dimethylcumene, 3,4-dimethylcumene and 2,6-dimethylcumene, ethylcumene, sec-butylbenzene, diisopropylbenzenes such as o-diisopropylbenzene, m-diisopropylbenzene, p-diisopropylbenzene and a mixture of diisopropylbenzene isomers, or 3,5-diisopropyltoluene. Among these aromatic compounds having a secondary alkyl group, there are preferably used aromatic compounds having a secondary alkyl group and a primary alkyl group, which are represented by the formula:

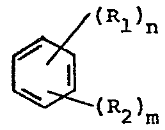

wherein $R_1$ stands for a secondary alkyl group having 3 to 4 carbon atoms, $R_2$ stands for a primary alkyl group having up to 4 carbon atoms, and n and m are either 1 or 2 with the proviso that the sum of n and m does not exceed 3.

Aromatic compounds having a secondary alkyl group and a methyl group are especially preferred. Of these, cymene, dimethylcumene and diisopropyltoluene are particularly preferred because their oxidation rates can be remarkably improved and the corresponding tertiary aromatic hydroperoxide may be prepared selectively in a high yield.

In the process of the present invention, the liquid phase catalytic oxidation of the aromatic compound having a secondary alkyl group is carried out in the presence of a basic aqueous solution. The base in this basic aqueous solution may be an alkali metal-containing base such as lithium hydroxide, sodium hydroxide, potassium hydroxide, lithium carbonate, sodium carbonate, potassium carbonate, sodium bicarbonate, potassium bicarbonate, sodium acetate, potassium acetate, sodium propionate, potassium propionate, sodium benzoate, sodium toluylate or sodium cuminate, or an alkaline earth metal-containing base such as magnesium hydroxide or barium hydroxide. Such a base is used in the form of an aqueous solution.

The pH value of the basic aqueous solution must be maintained at 7.6 to 10 during the catalytic oxidation reaction. In order to increase the oxidation rate and improve selectivity, it is preferred that the pH value of the basic aqueous solution be maintained at 7.7 to 8.7. If the pH value of the basic aqueous solution is lower than 7.6 or higher than 10 during the catalytic oxidation reaction, the oxidation rate is noticeably reduced and the selectivity to the tertiary aromatic hydroperoxide is markedly lowered. Moreover, if the pH value of the basic aqueous solution is higher than 10, the copper compound catalyst is precipitated and the catalytic activity is thus lowered. The volume ratio of the basic aqueous solution to the oily phase containing the aromatic compound having a secondary alkyl group is ordinarily 0.01 to 1:1. When this volume ratio is in the range of from 0.02 to 0.5:1, the oxidation rate can be particularly increased and the selectivity to the tertiary aromatic hydroperoxide may be further improved.

In the process of the present invention, an inorganic salt or organic acid salt of copper must be used as the copper compound catalyst. When a copper chelate compound disclosed in Japanese Patent Application Laid-Open Specification No. 142527/75 is used as a catalyst, it is inactivated in the abovementioned pH range. On the other hand, by selecting and using an inorganic salt or organic acid salt of copper, the activity of the catalyst can be maintained at a high level even when the pH value of the basic aqueous phase is within the above mentioned range, and the oxidation rate can be increased and the selectivity to the aromatic hydroperoxide can be improved.

The inorganic salt of copper used in the present invention may be, for example, copper (I) chloride, copper (II) chloride, copper (I) fluoride, copper (II) fluoride, copper (I) bromide, copper (II) bromide, copper (I) iodide, copper (II) iodide, copper (II) sulfate, copper (II) nitrate, copper (II) nitride, copper (II) perchlorate, copper (I) carbonate, copper (I) oxide, copper (II) oxide, copper (II) phosphate, copper (II) pyrophosphate, copper (II) potassium sulfate, copper (II) sulfide, copper (I) cyanide, copper (II) cyanide, copper thiocyanate, sodium copper (II) cyanide, potassium copper cyanide, copper (II) tungstate, copper (II) borate, copper (II) arsenite, copper (II) borofluoride, copper (II) chromate, copper (II) hydroxide, copper (II) selenate or copper (II) silicofluoride. The organic acid salt of copper may be, for example, copper (II) formate, copper (II) acetate, copper (II) propionate, copper (II) butyrate, copper (II) oxalate, copper (II) tartrate, copper (II) citrate, copper (II) laurate, copper (II) oleate, copper (II) salycilate, copper (II) stearate or copper (II) sulfamate. It is preferred that at least one of copper (II) nitrate, copper (II) perchlorate, copper (II) sulfate, copper (II) phosphate, copper (II) acetate, copper (II) benzoate or copper (II) citrate be used as the copper compound catalyst.

The liquid phase catalytic oxidation reaction is carried out in the presence of the copper compound catalyst. This catalyst should be used in such an amount that the copper compound catalyst concentration in the liquid phase, that is the oily phase containing the starting aromatic compound and the formed aromatic hydroperoxide and the basic aqueous phase, is 0.005 to 10 ppm, preferably 0.02 to 5 ppm, especially preferably 0.05 to 3 ppm, as the copper atom. If the copper compound catalyst is used in an amount larger than 10 ppm as the copper atom, decomposition of the aromatic hydroperoxide by the catalytic action of the copper catalyst becomes considerable and cannot be neglected, and the selectivity to the intended aromatic hydroperoxide is reduced. If the copper concentration is lower than 0.005 ppm, no substantial improvement in the oxidation rate or selectivity is attained.

Air or a gaseous mixture of oxygen and an inert gas haivng a variable oxygen concentration may be employed as the molecular oxygen-containing gas. Air is especially preferred.

The liquid phase catalytic oxidation of the starting aromatic compound is performed by contacting

an oily phase comprising the aromatic compound and which is being agitated with a basic aqueous phase with a molecular oxygen-containing gas. Ordinarily, the aromatic compound is used in a large excess, and oxidation is carried out in the absence of a particular reaction solvent. A solvent capable of dissolving therein the starting aromatic compound, which is insoluble in the basic aqueous solution phase and inactive to oxidation, may be used as the reaction solvent. For example, inert solvents such as benzene, chlorobenzene, dichlorobenzene and trifluoromethylbenzene may be used. When a solvent is used, the weight ratio of the solvent to the starting aromatic compound is ordinarily in the range of form 0.01 to 100:1. During or after oxidation, the oily phase contains the starting aromatic compound, the formed aromatic hydroperoxide, oil-soluble by-products and a minute amount of the copper compound catalyst as appropriate, and the solvent incorporated according to need. The basic aqueous phase contains a basic substance as mentioned above, water-soluble by-products, the organic acid salt formed as the by-product and the majority of the copper compound catalyst.

The liquid phase catalytic oxidation reaction is ordinarily carried out under heating. The reaction temperature is ordinarily from room temperature (15°C) to 200°C, preferably 60 to 150°C. The reaction pressure is preferably $1.99 \times 10^5$ to $5.00 \times 10^6$ Pa (1 to 50 Kg/cm$^2$-G). The reaction is carried out under agitation. Especially good results are obtained when the oily phase containing the starting aromatic compound and the basic aqueous solution phase are mixed under agitation, preferably so that a water-in-oil suspension or emulsion is formed, and the two liquid phases are sufficiently contacted with molecular oxygen-containing gas. For this purpose a perforated plate or agitator may be used. When a draft tube or the like is disposed in the vessel in which oxidation occurs, contact between the two liquid phases and the molecular oxygen-containing gas can be improved. The liquid phase catalytic oxidation reaction can be carried out in the presence of a radical initiator. The process may be effected in a continuous, semi-continuous or batchwise fashion. If the reaction mixture is allowed to stand after the reaction, it separates into the two liquid phases, the oily phase and the basic aqueous phase. The basic aqueous phase may be recycled to the reaction system and used again. If solvent is contained in the oily phase, this solvent is removed by distillation, and the residual mixture is supplied to a further stage in which the aromatic hydroperoxide that has been obtained is subjected to acid decomposition directly or after any unreacted starting compound has been distilled off, to obtain the corresponding phenol.

The following Examples illustrate the present invention.

### Example 1 to 6

A stainless steel continuous oxidation reaction vessel having a capacity of $7 \times 10^{-3} m^3$ (7 liters) and equipped with a reflux cooler disposed in the upper portion of the vessel to condense the vapor carried away by excessive air, a pipe to introduce air into the lower portion of the vessel, a pipe for introducing the starting material, catalyst and basic aqueous solution, a pipe for withdrawal of the liquid reaction mixture and an agitator was charged with a mixture of m- and p-cymenes, an aqueous solution of copper (II) sulfate and a 4% aqueous solution of sodium carbonate, which were supplied at predetermined flow rates. Continuous oxidation was carried out while blowing air at a predetermined rate into the reaction vessel until the concentration of cymene hydroperoxide (hereinafter referred to as "CyHP") in the liquid reaction mixture reached a stationary level. The oxidation product was subjected to oil-water separation. CyHP was analyzed according to the customary iodometry method. Unreacted cymene and oxidation by-products were analyzed by gas chromatography.

The conditions adopted for the oxidation process were: reaction temperature of 120°C, reaction pressure of $5.92 \times 10^5$ Pa (5 Kg/cm$^2$-G), agitation rate of 750 rpm, oil phase/aqueous phase volume ratio of 9/1 and copper atom concentration of 0.07, 0.18, 0.44, 0.63, 1.03 or 2.89 ppm based on the liquid phase (oily phase + aqueous phase). The CyHP concentration in the oily phase after oxidation was adjusted to 14 to 15% by weight by controlling the feed rate of cymene. The pH of the aqueous phase was adjusted to 7.8 to 8.1 by controlling the feed rate of the aqueous solution of sodium carbonate. The conditions of agitation in the reaction vessel were such that a water-in-oil emulsion formed. The results are shown in Table 1.

TABLE 1

| Example No. | Copper Atom Concentration (ppm) | CyHP Accumulation Rate $Kg/m^3.s$ (g/l.hr) *1 | Oxidation Efficiency (mol%) *2 |
|---|---|---|---|
| 1 | 0.07 | $1.33\times10^{-2}$ (48) | 79 |
| 2 | 0.18 | $1.47\times10^{-2}$ (53) | 80 |
| 3 | 0.44 | $1.50\times10^{-2}$ (54) | 78 |
| 4 | 0.63 | $1.61\times10^{-2}$ (58) | 75 |
| 5 | 1.03 | $1.64\times10^{-2}$ (59) | 76 |
| 6 | 2.89 | $1.72\times10^{-2}$ (62) | 77 |

Note

*1: the amount of cymene hydroperoxide accumulated per unit volume of the reaction vessel for unit time.

*2: the ratio (%) of the moles of formed cymene hydroperoxide to the moles of consumed starting cymene.

Comparative Example 1

By using the same continuous oxidation vessel as used in Examples 1 to 6, continuous oxidation of a mixture of m- and p-cymenes was carried out under conditions of a reaction temperature of 120°C, a reaction pressure of $5.92 \times 10^5$ Pa (5 $Kg/cm^2$-G), an agitation rate of 750 rpm, an aqueous phase pH of 8.0 and an oily phase/aqueous phase volume ratio of 9/1 without using the copper catalyst. The CyHP concentration in the liquid reaction mixture was maintained at 14.7% by weight by adjusting the residence time of starting cymene. The CyHP accumulation rate and oxidation efficiency were $7.5 \times 10^{-3}$ $Kg/m^3.s$ (27 g/l.hr) and 69 mol%, respectively.

Comparative Example 2

By using the same continuous oxidation vessel as used in Examples 1 to 6, continuous oxidation of a mixture of m- and p-cymenes was carried out under conditions of a reaction temperature 120°C, a reaction pressure of $5.92 \times 10^5$ Pa (5 $Kg/cm^2$-G), an agitation rate of 750 rpm, an aqueous phase pH of 8.0 and an oily phase/aqueous phase volume ratio of 9/1 by adding a copper sulfate catalyst at a copper atom concentration of 12 ppm based on the entire liquid phase, while maintaining the CyHP concentration in the liquid reaction mixture at 14.5% by weight. The CyHP accumulation rate and oxidation efficiency were $8.9 \times 10^{-3}$ $Kg/m^3.s$ (32 g/l.hr) and 42 mol%, respectively.

Examples 7 to 10

By using the same continuous oxidation vessel as used in Examples 1 to 6, continuous oxidation of a mixture of m- and p-cymenes was carried out under conditions of a reaction temperature of 120°C, a reaction pressure of $5.92 \times 10^5$ Pa (5 $Kg/cm^2$-G) and an agitation rate of 750 rpm in the presence of a copper sulfate catalyst at a copper atom concentration of 0.15 ppm based on the liquid phase, while maintaining the CyHP concentration in the liquid reaction mixture at 14 to 15% by weight by controlling the residence time of starting cymene. The pH value of the aqueous phase was set at 7.7, 7.8, 8.1 or 8.5 by changing the feed rate of the 4% aqueous solution of sodium carbonate. The results are shown in Table 2.

TABLE 2

| Example No. | pH Value of Aqueous Phase | CyHP Accumulation Rate Kg/cm$^3$.s (g/l.hr) | Oxidation Efficiency (mol%) |
|---|---|---|---|
| 7 | 7.7 | $1.42 \times 10^{-2}$ (51) | 78 |
| 8 | 7.8 | $1.47 \times 10^{-2}$ (53) | 80 |
| 9 | 8.1 | $1.44 \times 10^{-2}$ (52) | 77 |
| 10 | 8.5 | $1.42 \times 10^{-2}$ (51) | 78 |

Comparative Examples 3 and 4

By using the same continuous oxidation vessel as used in Examples 1 to 6, continuous oxidation of a mixture of m- and p-cymenes was carried out under the same conditions as in Examples 7 to 10 except that the pH value of the aqueous phase was changed to 7.3 or 10.6. When the pH value of the aqueous phase was 7.3, the CyHP accumulation rate and oxidation efficiency were $10^{-2}$ Kg/m$^3$.s (36 g/l.hr) and 70 mol%, respectively, and when the pH value of the aqueous phase was 10.6, the CyHP accumulation rate and oxidation efficiency were $8.1 \times 10^{-3}$ Kg/m$^3$.s (29 g/l.hr) and 69 mol%, respectively.

Examples 11 to 13

By using the same continuous oxidation vessel as used in Examples 1 to 6, continuous oxidation of a mixture of m- and p-cymenes was carried out under conditions of a reaction temperature of 120°C, a reaction pressure of $5.92 \times 10^5$ Pa (5 Kg/cm$^2$-G), an agitation rate of 750 rpm and an aqueous phase pH value of 7.9 in the presence of a copper sulfate catalyst at a copper atom concentration of 0.21 ppm based on the liquid phase. The volume ratio of the aqueous phase to the oily phase was adjusted to 8/92, 19/81 or 31/69. The results are shown in Table 3.

TABLE 3

| Example No. | Aqueous Phase/ Oily Phase Volume Ratio | CyHP Accumulation Rate Kg/m$^3$.s (g/l.hr) | Oxidation Efficiency (mol%) |
|---|---|---|---|
| 11 | 8/92 | $1.44 \times 10^{-2}$ (52) | 79 |
| 12 | 19/81 | $1.47 \times 10^{-2}$ (53) | 80 |
| 13 | 31/69 | $1.44 \times 10^{-2}$ (52) | 78 |

Examples 14 to 17

By using the same continuous oxidation vessel as used in Examples 1 to 6, continuous oxidation of a mixture of m- and p-cymenes was carried out under the same conditions as in Example 2 except that copper (II) nitrate, copper (II) chloride, copper (II) acetate or copper (II) stearate was used as the catalyst instead of copper (II) sulfate. The results are shown in Table 4.

TABLE 4

| Example No. | Catalyst | CyHP Accumulation Rate Kg/m³.s (g/l.hr) | Oxidation Efficiency (mol%) |
|---|---|---|---|
| 14 | copper (II) nitrate | $1.44\times10^{-2}$ (52) | 79 |
| 15 | copper (II) chloride | $1.47\times10^{-2}$ (53) | 78 |
| 16 | copper (II) acetate | $1.50\times10^{-2}$ (54) | 80 |
| 17 | copper (II) stearate | $1.44\times10^{-2}$ (52) | 79 |

**Claims**

1. A process for the preparation of an aromatic tertiary hydroperoxide by contacting an oily liquid phase comprising an aromatic compound having a secondary alkyl group, which phase is being agitated with a basic aqueous liquid phase, with a molecular oxygen-containing gas in the presence of a metal compound catalyst, thereby oxidizing the aromatic compound to a corresponding aromatic tertiary hydroperoxide, characterised in that the metal compound catalyst is a copper compound catalyst composed of an inorganic salt or organic acid salt of copper and in that during oxidation the total concentration of the copper compound catalyst in the two phases is maintained at from 0.005 to 10 ppm, calculated on the basis of copper in the catalyst, and the pH value of the basic aqueous phase is maintained at from 7.6 to 10.

2. A process according to claim 1, wherein the volume ratio of the basic aqueous solution phase to the oily phase is from 0.01 to 1:1.

3. A process according to claim 1 or 2, wherein the concentration of the copper compound catalyst in the two phases is maintained at from 0.02 to 5 ppm.

4. A process according to any one of claims 1 to 3, wherein the pH value of the basic aqueous phase is maintained at from 7.7 to 8.7.

5. A process according to any one of claims 1 to 4, wherein the aromatic compound is an aromatic compound having a secondary alkyl group and a methyl group.

6. A process according to claim 5, wherein the aromatic compound is cymene, dimethylcumene or diisopropyltoluene.

7. A process according to any one of claims 1 to 6, wherein oxidation is carried out at a temperature of from 60 to 150°C under a pressure of from $1.99 \times 10^5$ to $5.00 \times 10^6$ Pa.

8. A phenol which has been prepared from an aromatic tertiary hydroperoxide, prepared by a process as claimed in any one of the preceding claims, by acid decomposition.

**Revendications**

1. Procédé de préparation d'un hydroperoxide tertiaire aromatique dans lequel on met en contact une phase huileuse liquide comprenant un composé aromatique ayant un groupement alkyle secondaire, on agite ladite phase avec une phase liquide aqueuse basique, avec un gaz contenant de l'oxygène moléculaire en présence d'un dérivé métallique catalyseur, de façon à oxyder le composé aromatique en un hydroperoxide tertiaire aromatique correspondant, caractérisé par le fait que le dérivé métallique catalyseur est un catalyseur dérivé du cuivre composé d'un sel minéral ou d'un sel d'acide organique de cuivre, et par le fait que pendant l'oxydation la concentration totale du dérive de cuivre catalyseur dans les deux phases est maintenue à une valeur de 0,005 à 10 ppm, calculée par rapport au cuivre présent dans le catalyseur, et la valeur du pH de la phase basique aqueuse est maintenue à une valeur de 7,6 à 10.

2. Procédé selon la revendication 1, caractérisé par le fait que le rapport du volume de la phase aqueuse basique au volume de la phase huileuse varie de 0,01 à 1:1.

3. Procédé selon la revendication 1 ou 2, caractérisé par le fait que la concentration du dérivé de cuivre catalyseur dans les deux phases est maintenue à une valeur de 0,02 à 5 ppm.

4. Procédé selon l'une quelconque des revendications 1 à 3, caractérisé par le fait que le pH de la phase aqueuse basique est maintenu à une valeur de 7,7 à 8,7.

5. Procédé selon l'une quelconque des revendications 1 à 4, caractérisé par le fait que le composé aromatique possède un groupement alkyle secondaire et un groupement méthyle.

6. Procédé selon la revendication 5, caractérisé par le fait que le composé aromatique est le cymène, le diméthylcumène ou le diisopropyltoluène.

7. Procédé selon l'une quelconque des revendications 1 à 6, caractérisé par le fait que l'oxydation est effectuée à une température de 60 à 150°C sous une pression de $1{,}99 \times 10^5$ à $5{,}00 \times 10^6$ Pa.

8. Phénol qui a été obtenu, au départ d'un hydroperoxide tertiaire aromatique préparé par un procédé tel que défini dans l'une quelconque des revendications précédentes, par décomposition acide.

**Patentansprüche**

1. Verfahren zur Herstellung eines aromatischen tertiären Hydroperoxids, bei dem eine ölige Flüssigphase, die eine aromatische Verbindung mit einer sekundären Alkylgruppe enthält und die mit einer basischen wässrigen Flüssigphase gerührt wird, mit einem molekularen Sauerstoff enthaltenden Gas in der Gegenwart eines eine Metallverbindung enthaltenden Katalysators zusammengebracht wird, wobei die aromatische Verbindung zu dem entsprechenden aromatischen tertiären Hydroperoxid oxidiert wird, dadurch gekennzeichnet, dass als Katalysator eine Kupferverbindung verwendet wird, die aus einem anorganischen oder einem sauren organischen Kupfersalz gebildet wird und dass während der Oxydation die Gesamtkonzentration der Kupferverbindung in den beiden Phasen zwischen 0.005 und 10 ppm, bezogen auf den Kupfergehalt im Katalysator und der pH-Wert der basischen Flüssigphase zwischen 7.6 und 10 gehalten wird.

2. Verfahren nach Anspruch 1, dadurch gekennzeichnet, dass das Volumenverhältnis zwischen der basischen wässrigen Flüssigphase und der öligen Phase zwischen 0.01 und 1:1 liegt.

3. Verfahren nach einem oder beiden der vorhergehenden Ansprüche, dadurch gekennzeichnet, dass die Konzentration der Kupferverbindung in den beiden Phasen zwischen 0.02 und 5 ppm gehalten wird.

4. Verfahren nach einem der vorhergehenden Ansprüche, dadurch gekennzeichnet, dass der pH-Wert der basischen Flüssigphase zwischen 7.7 und 8.7 gehalten wird.

5. Verfahren nach einem der vorhergehenden Ansprüche, dadurch gekennzeichnet, dass eine aromatische Verbindung mit einer sekundären Alkyl- und einer Methylgruppe verwendet wird.

6. Verfahren nach Anspruch 5, dadurch gekennzeichnet, dass als aromatische Verbindung Zymol, Dimethylcumol oder Diisopropyltoluol verwendet wird.

7. Verfahren nach einem der vorhergehenden Ansprüche, dadurch gekennzeichnet, dass die Oxydation bei Temperaturen zwischen 60 und 150°C unter einem Druck zwischen $1.99 \times 10^5$ und $5.00 \times 10^6$ Pa durchgeführt wird.

8. Phenol hergestellt aus einem gemäss einem Verfahren nach einem der vorhergehenden Ansprüche erhaltenen aromatischen tertiären Hydroperoxid durch Säurespaltung.